# EUROPEAN PATENT APPLICATION

(11) **EP 2 980 078 A1**
(43) Date of publication of application: **03.02.2016**
(21) Application number: 14178988.3
(22) Date of filing: 29.07.2014
(51) Int. Cl.: C07D 231/14

(54) **Process for the preparation of pyrazole-4-carboxamides**

(71) Applicant: Solvay SA, 1120 Bruxelles (BE)
(72) Inventor: Braun, Max Josef, 30900 Wedemark (DE)
(74) Representative: Mross, Stefan P.M.

(57) **Abstract**

The invention concerns a process for the manufacture of pyrazole-4-carboxamides, in particular, of 3-difluoromethyl-1-methyl-1 H-pyrazole-4-carboxamides which are useful as pharmaceuticals and agrochemicals.

The carboxamides are prepared from the corresponding pyzole-4-carboxylic acid esters and appropriate amine in the presence of a Lewis acid. Alternatively, the reaction is performed in the presence of a Lewis acid and a base.

## Description

The invention concerns a process for the manufacture of 1H-pyrazole-4-carboxamides, in particular 3-difluoromethyl-1-methyl- 1H-pyrazole-4-carboxamides, which are useful as pharmaceuticals and agrochemicals.

Particular examples of 3-difluoromethyl-1-methyl- 1H-pyrazole-4-carboxamides are for instance Bixafen, Sedaxane, Isopyrazam and Fluxapyraxad.

Bixafen having the chemical name N-(3',4'-dichloro-5-fluoro-[1,1'-biphenyl]-2-yl)-3-(difluoro- methyl)-1-methyl- lH-pyrazole-4-carboxamide (CAS Number 581809-46-3) and its manufacturing process is described in WO 03/070705.

Sedaxane is a mixture of isomers N-(2-[1,1'-bicyclopropyl]-2-ylphenyl)-3-(difluoromethyl)-1-methyl-1H-pyrazole-4-carboxamide (CAS Number 874967-67-6). Sedaxane and its manufacturing process are for example described in WO 2006/015865 and WO 2006/015866.

Isopyrazam is a mixture of isomers of 3-(difluoromethyl)-1-methyl-N-[1,2,3,4-tetrahydro-9-isopropyl-1,4-methanonaphthalen-5-yl]pyrazole-4-carboxamide (CAS Number 881685-58-1). Isopyrazam and its manufacturing process are described in WO 2004/035589.

Fluxapyroxad having the chemical name 3-(Difluoromethyl)-l-methyl-N-(3',4',5'-trifluorobiphenyl- 2-yl)-1H-pyrazole-4-carboxamide and its manufacturing process is described in WO 2006/087343.

1H-pyrazole-4-carboxamides are generally obtained by reacting the corresponding 4-carboxylic acid pyrazole or the activated form of said carboxylic acid, for example an acid chloride, with an appropriate amine, see for example WO 03/070705 (EP1490342), WO 2005/123690, WO 2006/087343 or WO 2007/009717.

US5556987 describes a process for the production of 5-hydroxypyrazolecarboxamides which is catalysed with a Lewis acid such as AlCl₃.

It is an object of the present invention to provide a process for the synthesis of 1H-pyrazole-4-carboxamides which allows, in particular, in an economically manner for high yield, high purity, and high efficiency for the manufacture of the target product. The process can have environmental benefits.

The invention consequently relates to a process for the manufacture of for the manufacture of compounds of formula (I) 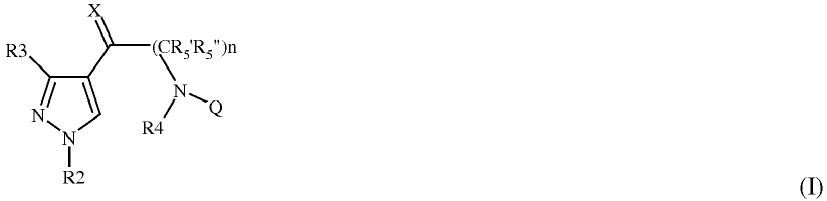 wherein
- R2 is H or an organic residue
- R3 is H, a halogen, an aliphatic, which also comprises alicyclic, heteroaliphatic, which also comprises heterocyclic, heteroaromatic or aromatic group, in particular an alkyl group having from 1 to 12 carbon atoms, a halogenated alkyl group having from 1 to 12 carbon atoms, an aralkyl group, an aryl group
- R4 is H, a halogen, an aliphatic, which also comprises alicyclic, heteroaliphatic, which also comprises heterocyclic, heteroaromatic or aromatic group, in particular an alkyl group having from 1 to 12 carbon atoms, a halogenated alkyl group having from 1 to 12 carbon atoms, an aralkyl group, an aryl group,
- R₅' and R₅" are each independently selected from H, a halogen, an aliphatic, which also comprises alicyclic, heteroaliphatic, which also comprises heterocyclic, heteroaromatic or aromatic group, in particular an alkyl group having from 1 to 12 carbon atoms, a halogenated alkyl group having from 1 to 12 carbon atoms, an aralkyl group, an aryl group,
- n = 0 to 10
- X is oxygen or sulfur
- Q is an alicyclic, heterocyclic, heteroaromatic or aromatic group, in particular a group of any of formulae (Q1) to (Q38) herein below:

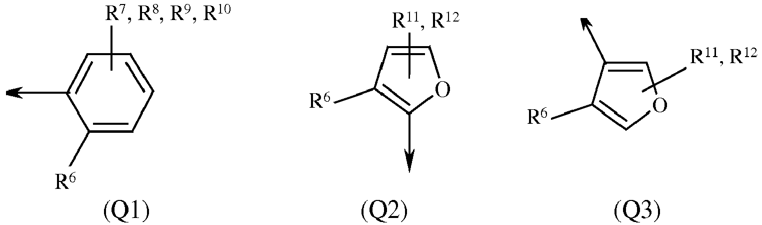

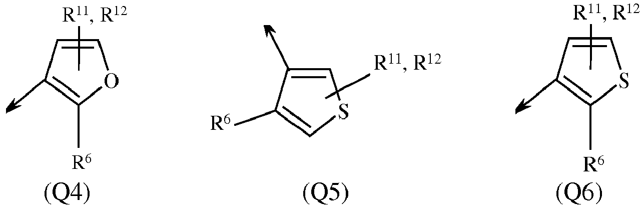

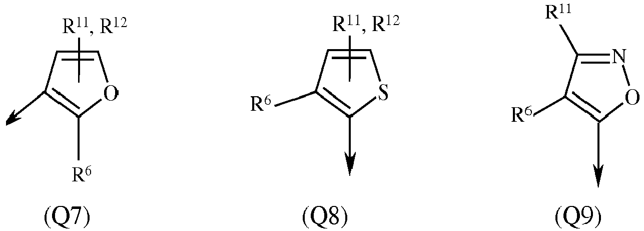

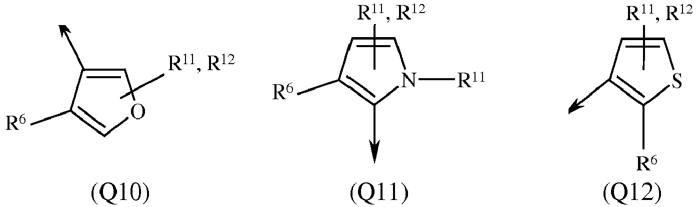

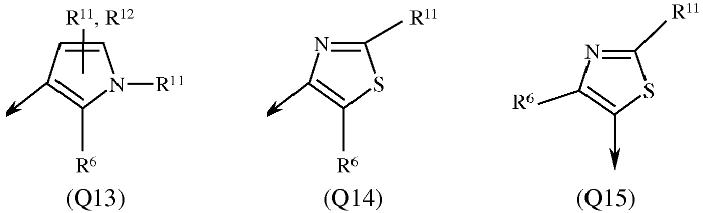

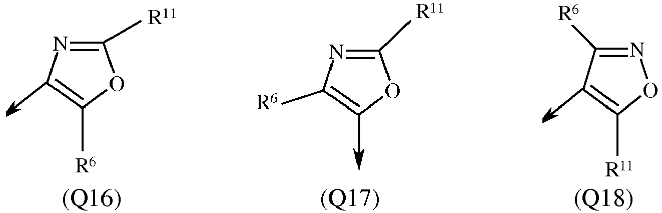

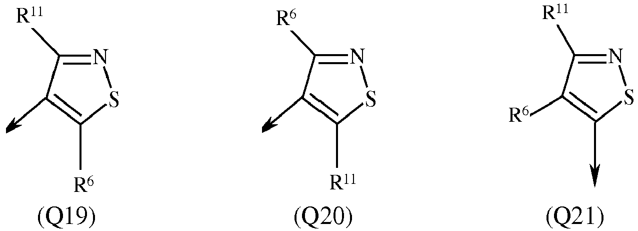

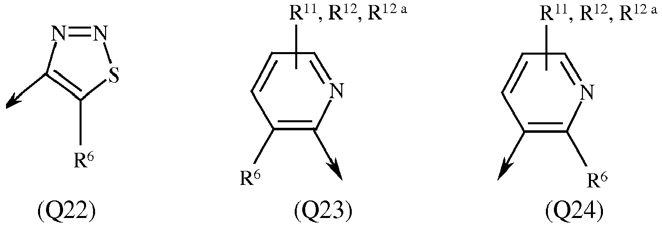

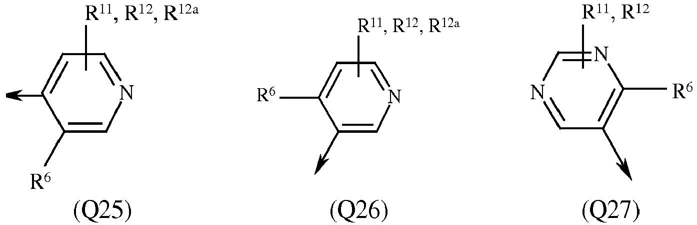

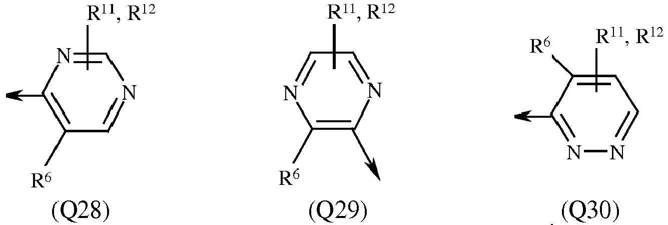

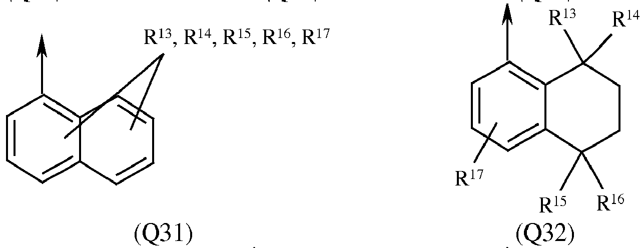

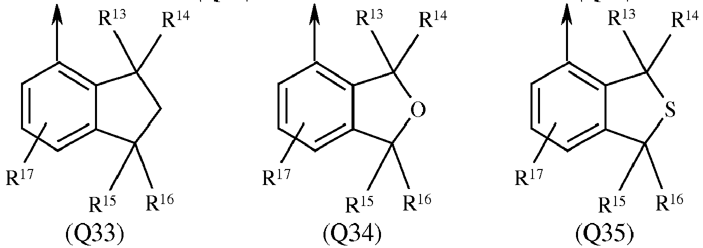

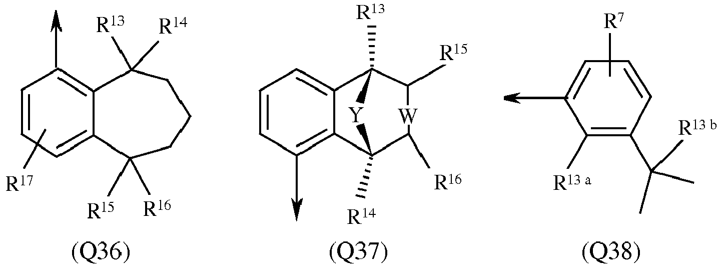

wherein R⁶ is a hydrogen, C₁₋₁₂ alkyl, C₂₋₁₂ alkenyl or C₂₋₁₂ alkynyl group, which may be substituted by 1 to 6 substituents, each substituent independently selected from halogen, cyano, C₁₋₄ alkoxy, C₁₋₄ thioalkyl, COO-C₁₋₄ alkyl, =N-OH, =N-0-(C₁₋₄ alkyl), C₃₋₈ cycloalkyl, which may itself be substituted by 1 to 3 substituents, each independently selected from C₁₋₄ alkyl, halogen, C₁₋₄ alkoxy and C₁₋₄ haloalkoxy, and C₄₋₈ cycloalkenyl, which may itself be substituted by 1 to 3 substituents, each independently selected from C₁₋₄ alkyl, halogen, C₁₋₄ alkoxy and C₁₋₄ haloalkoxy ;
or R⁶ is a C₃₋₈ cycloalkyl, C₄₋₈ cycloalkenyl or C₅₋₈ cycloalkadienyl group, which may be substituted by 1 to 3 substituents, each independently selected from halogen, C₁₋₄ alkyl, C₁₋₄ haloalkyl, C₁₋₄ alkoxy, C₁₋₄ haloalkoxy, C₁₋₄ thioalkyl, C₃₋₆ cycloalkyl, which may itself be substituted by 1 to 3 substituents, each independently selected from C₁₋₄ alkyl, halogen, C₁₋₄ alkoxy and C₁₋₄ haloalkoxy, and phenyl, which may itself be substituted by 1 to 5 independently selected halogen atoms ;
or R⁶ is a C₆₋₁₂ bicycloalkyl, C₆₋₁₂ bicycloalkenyl or C₆₋₁₂ bicycloalkadienyl group, which may be substituted by 1 to 3 substituents, each independently selected from halogen, C₁₋₄ alkyl and C₁₋₄ haloalkyl ;
or R⁶ is phenyl, which may be substituted by 1 to 3 substituents, each independently selected from halogen, cyano, nitro, C₁₋₄ alkyl, C₁₋₄ haloalkyl, C₁₋₄ alkoxy, C₁₋₄ alkylthio, C₁₋₄ haloalkoxy, C₁₋₄ haloalkylthio, C(H)=N-OH, C(H)=N-O(C₁₋₆ alkyl), C(C₁₋₆ alkyl)=N-OH, C(C₁₋₆ alkyl)=N-O-(C₁₋₆ alkyl), (Z)C≡CR, (Z)ₙCR²⁸ =CR²⁶ R²⁷, phenyl, which may itself be substituted by 1 to 3 substituents, each independently selected from halogen, cyano, nitro, C₁₋₄ alkyl, C₁₋₄ haloalkyl, C₁₋₄ alkoxy, C₁₋₄ haloalkoxy, C₁₋₄ haloalkylthio, C(H)=N-OH, C(H)=N-O(C₁₋₆ alkyl), C(C₁₋₆ alkyl)=N-OH and C(C₁₋₆ alkyl)=N-O-(C₁₋₆ alkyl), and thienyl, which may itself be substituted by 1 to 3 substituents, each independently selected from halogen, cyano, nitro, C₁₋₄ alkyl, C₁₋₄ haloalkyl, C₁₋₄ alkoxy, C₁₋₄ haloalkoxy, C₁₋₄ haloalkylthio, C(H)=N-OH, C(H)=N-O(C₁₋₆ alkyl), C(C₁₋₆ alkyl)=N-OH and C(C₁₋₆ alkyl)=N-O-(C₁₋₆ alkyl) ;
or R⁶ is a 5-6 membered heterocyclic ring, wherein the heterocyclic ring contains 1 to 3 heteroatoms, each heteroatom independently chosen from oxygen, sulphur and nitrogen, wherein the heterocyclic ring may be substituted 1 to 3 substituents, each independently selected from halogen, cyano, nitro, C₁₋₄ alkyl, C₁₋₄ haloalkyl, C₁₋₄ alkoxy, C₁₋₄ alkylthio, C₁₋₄alkylthio, C₁₋₄ haloalkoxy, C(H)=N-O-(C₁₋₆ alkyl) and C(C₁₋₆ alkyl)=N-O-(C₁₋₆ alkyl), C₂₋₅ alkenyl, C₂₋₅ alkynyl, CHO, COOC₁-C₆ alkyl, CrC₄ alkoxy-C₁-C₄ alkyl, CrC₄ haloalkoxy-C₁-C₄ alkyl, (Z)_{P}C≡CR, (Z)ₙCR²⁸ =CR²⁶ R²⁷, phenyl, which may itself be substituted by 1 to 3 substituents, each independently selected from halogen, cyano, nitro, C₁₋₄ alkyl, C₁₋₄ haloalkyl, C₁₋₄ alkoxy, C₁₋₄ haloalkoxy, C₁₋₄ haloalkylthio, C(H)=N-OH, C(H)=N-O(C₁₋₆ alkyl), C(C₁₋₆ alkyl)=N-OH and C(C₁₋₆ alkyl)=N-O-(C₁₋₆ alkyl), and thienyl, which may itself be substituted by 1 to 3 substituents, each independently selected from halogen, cyano, nitro, C₁₋₄ alkyl, C₁₋₄ haloalkyl, C₁₋₄ alkoxy, C₁₋₄ haloalkoxy, C₁₋₄ haloalkylthio, C(H)=N-OH, C(H)=N-O(C₁₋₆ alkyl), C(C₁₋₆ alkyl)=N-OH and C(C₁₋₆ alkyl)=N-O-(C₁₋₆ alkyl), and wherein two substituents on adjacent carbon atoms of the 5-6 membered heterocyclic ring together may form a group -CR^{6a} -CR^{6a} =CR^{6a} -CR^{6a} -, wherein each R^{6a} independently is selected from hydrogen, halogen, cyano, nitro, C₁₋₄ alkyl, C₁₋₄ haloalkyl, C₁₋₄ alkoxy, C₁₋₄ haloalkoxy, C₁₋₄ haloalkylthio, C(H)=N-OH, C(H)=N-O(C₁₋₆ alkyl), C(C₁₋₆ alkyl)=N-OH and C(C₁₋₆ alkyl)=N-O-(C₁₋₆ alkyl) ;
or R⁶ is an aliphatic saturated or unsaturated group containing 3 to 13 carbon atoms and at least one silicon atom, wherein the aliphatic group may contain 1 to 3 heteroatoms, each heteroatom independently selected from oxygen, nitrogen and sulphur, and wherein the aliphatic group may be substituted by 1 to 4 independently selected halogen atoms ;
or R⁶1s (CR^{a} R^{b})ₘ-Cy-(CR^{c} R^{d})ₙ-Y₁;
or R⁶ is C₁₋₆ alkoxy, C₁₋₆ haloalkoxy, C₂₋₆ alkenyloxy, C₂₋₆ haloalkenyloxy, C₂₋₆ alkinyloxy, C₃₋₆ cycloalkyloxy, C₁₋₄ alkyl-C₃₋₇ cycloalkyloxy, C₅₋₇ cycloalkenyloxy or C₁₋₄ alkyl-C₅₋₇ cycloalkenyloxy ;
Z is C₁₋₄ alkylene ;
p is 0 or 1;
R²⁵ is hydrogen, halogen, C₁₋₄ alkyl, C₁₋₄ haloalkyl, C₁₋₄ alkoxy (C₁₋₄) alkyl,
C₁₋₄ haloalkoxy (C₁₋₄) alkyl or Si(C₁₋₄ alkyl)₃;
R²⁶ and R²⁷ are each, independently, hydrogen, halogen, C₁₋₄ alkyl or
C₁₋₄ haloalkyl;
R²⁵ is hydrogen, C₁₋₄ alkyl or C₁₋₄ haloalkyl;
R^{a}, R^{b}, R^{c} and R^{d} are each, independently, hydrogen or a C₁₋₄ alkyl group, which may substituted by 1 to 6 substituents, each substituent independently selected from halogen, hydroxy, cyano, carboxyl, methoxycarbonyl, ethoxycarbonyl, methoxy, ethoxy, methylsulfonyl, ethylsulfonyl, difluoromethoxy, trifluoromethoxy, trifluoromethylthio and trifluorothiomethoxy ; Cy is a carbocyclic or heterocyclic 3-7 membered ring, which may be saturated, unsaturated or aromatic and which may contain a silicon atom as a ring member, wherein (CR^{a} R^{b})ₘ and (CR^{c}R^{d})ₙ may be bound either to the same carbon or silicon atom of Cy or to different atoms separated by 1, 2 or 3 ring members, wherein the carbocyclic or heterocyclic 3-7 membered ring may substituted by 1 to 6 substituents, each substituent independently selected from halogen, C₁₋₄ alkyl, C₂₋₄ alkenyl, C₁₋₄ haloalkyl, C₁₋₄alkoxy and halo-C₁₋₄ alkoxy ;
Y₁ is Si(Oₚ₁Z¹)(O_{q}Z²)(O≤Z³) and provided that Cy contains a silicon atom as a ring member then Y₁ may also be hydrogen ;
Z¹ and Z² are independently methyl or ethyl;
Z³ is a C₁₋₄ alkyl or a C₂₋₄ alkenyl group, which may be interrupted by one heteroatom selected from O, S and N, and wherein the C₁₋₄ alkyl or C₂₋₄ alkenyl group may be substituted by 1 to 3 independently selected halogen atoms ; m and n are each independently 0, 1, 2 or 3 ;
p₁, q and s are each independently 0 or 1 ;
R⁷, R⁸, R⁹, R¹⁰, R¹¹, R¹² and R^{12a} are each, independently, hydrogen, halogen, cyano, nitro, C₁₋₄ alkyl, C₁₋₄ haloalkyl, C₁₋₄ alkoxy, C₁₋₄ haloalkoxy, C₁₋₄ thioalkyl or C₁₋₄ thiohaloalkyl ;
R¹³, R¹⁴, R¹⁵, R¹⁶ and R¹⁷ are each, independently, hydrogen, halogen, cyano, nitro, C₁₋₄ alkyl, C(O)CH₃, C₁₋₄ haloalkyl, C₁₋₄ alkoxy, C₁₋₄ haloalkoxy, C₁₋₄ thioalkyl, C₁₋₄ thiohaloalkyl, hydroxymethyl or C₁₋₄ alkoxymethyl ;
W is a single or a double bond ; and
Y is O, N(R¹⁸), S or (CR₁₉R₂₀)(CR₂₁R₂₂)ₘ₁(CR₂₃R₂₄)ₙ₁ ;
R¹⁸ is hydrogen, C₁₋₄ alkyl, formyl, C₁₋₄ alkoxy(C₁₋₄)alkyl, C(=O)C₁₋₄ alkyl, which may be substituted by halogen or C₁₋₄-alkoxy, or C(=O)O-C₁₋₆ alkyl, which may be substituted by halogen, C₁₋₄ alkoxy or CN ; R¹⁹, R²⁰, R²¹, R²², R²³ and R²⁴ are each independently hydrogen, halogen, hydroxy, C₁₋₄ alkoxy, C₁₋₆ alkyl, which may be substituted by 1 to 3 substituents selected from halogen, hydroxy, =O, C₁₋₄ alkoxy, O-C(O)-C₁₋₄ alkyl, phenyl, naphthyl, anthracyl, fluorenyl, indanyl or a 3-7 membered carbocyclic ring (which itself may be substituted by 1 to 3 methyl groups), C₁₋₆ alkenyl, which may be substituted by 1 to 3 substituents selected from halogen, hydroxy, =O, C₁₋₄ alkoxy, O-C(O)-C₁₋₄ alkyl, phenyl, naphthyl, anthracyl, fluorenyl, indanyl or a 3-7 membered carbocyclic ring (which itself may be substituted by 1 to 3 methyl groups), or a 3- 7 membered carbocyclic ring, which may contain 1 heteroatom selected from nitrogen and oxygen, and wherein the 3-7 membered carbocyclic ring may be substituted by 1 to 3 methyl groups ; or R¹⁹, R²⁰ together with the carbon atom to which they are attached form a carbonyl-group, a 3-5 membered carbocyclic ring, which may be substituted by 1 to 3 methyl groups, C₁₋₆ alkylidene, which may be substituted by 1 to 3 methyl groups, or C₃₋₆ cycloalkylidene, which may be substituted by 1 to 3 methyl groups ;
m₁ is 0 or 1;
n₁ is 0 or 1;
R^{13a} is a C₁-C₄ alkyl, C₂-C₄ alkenyl or C₂-C₄ alkynyl group, which may be substituted by 1 to 6 substituents, each substituent independently selected from halogen, hydroxy, cyano, C₁₋₄ alkoxycarbonyl, formyl, nitro, C₁-C₄ alkoxy, C₁-C₄ haloalkoxy, C₁-C₄ alkylthio, C₁-C₄ haloalkylthio, HC(OR²⁹)=N-
and R³⁰R³¹NN=C(H)- ;
R²⁹, R³⁰ and R³¹ independently of one another are hydrogen or C₁-C₄ alkyl; R^{13b} is a C₁-C₆ alkyl group, which may be substituted by 1 to 6 substituents, each substituent independently selected from halogen, hydroxy, cyano, C₁₋₄ alkoxycarbonyl, formyl, nitro, C₁-C₄ alkoxy, C₁-C₄ haloalkoxy,
C₁-C₄ alkylthio, C₁-C₄ haloalkylthio, HC(OR³²)=N- and R³³R³⁴NN=C(H)- ; R³², R³³ and R²⁴ independently of one another are hydrogen or C₁-C₄ alkyl; R^{13c} is hydrogen or halogen ; and tautomers/isomers/enantiomers of these compounds
which comprises reacting a compound of formula (II) 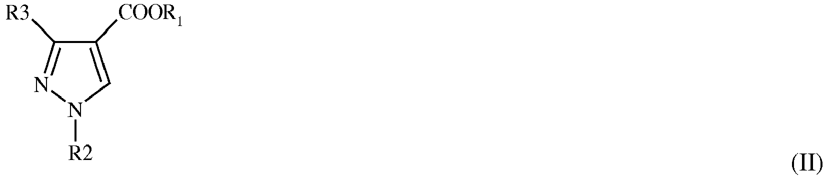 wherein :
- R1 is an organic residue

R2 and R3 are as defined above, with an amine of formula (III) :
QNHR4 (III) wherein Q is as defined above, in the presence of a Lewis acid.

It has been found, surprisingly, that when a compound of formula (II) and an amine of formula (III) are reacted in the presence of a Lewis acid, a highly efficient production of the desired carboxamide of formula (I) is obtained. The Lewis acid for use in this invention is now explained.

The Lewis acid, which serves as an electron acceptor, usually comprises a typical element, such as boron, aluminum, silicon, or tin, or a transition metal element belonging to the fourth period, such as titanium, iron, nickel, copper, or zinc, as the central element. Aluminum as central element is preferred.

The ligands of the Lewis acid central element may be ions of a halide such as a chloride or fluoride or ions of an alkoxide such as an ethoxide, propoxide, or butoxide. Alkyl groups, such as methyl, ethyl, n-propyl, i-propyl and all isomeric forms of butyl; methyl is preferred. Lewis acids, specifically AlCl₃ and AlMe₃, are well known in the art and commercially available. In the Lewis acid, the ligands bonded to the central element may be of one kind or may consist of a combination of two or more kinds. In the case of using a Lewis acid having two or more kinds of ligands, such a Lewis acid may be prepared beforehand or may be synthesized in situ through exchange reaction by a well-known method (see Kaoru Fuji and Manabu Noide, Yuki Gosei Kagaku (Organic Synthesis Chemistry), 42, 194 (1984)).

Specific examples of the Lewis acid for use in this invention include boron trihalides such as boron trifluoride, boron trichloride, and boron tribromide, aluminum trihalides such as aluminum chloride and aluminum bromide, tin tetrahalides such as tin tetrachloride, tin dihalides such as tin dichloride, titanium tetrahalides such as titanium tetrachloride, titanium trihalides such as titanium trichloride, titanium alkoxides such as titanium isopropoxide, iron dihalides such as iron dichloride, iron trihalides such as iron trichloride, nickel dihalides such as nickel dichloride, and zinc halides such as zinc chloride and zinc bromide.

More preferred Lewis acids are boron trihalides, aluminum trihalides, tin tetrahalides, titanium tetrahalides, titanium alkoxides, iron trihalides, and zinc halides. Further preferred of these are iron trichloride, aluminum chloride, titanium isopropoxide, titanium tetrachloride, and zinc chloride, with aluminum chloride being the most preferred, especially for the process according to the second embodiment. Another most preferred Lewis acid according to the present invention is AlMe₃.

In the first embodiment of the process according to the present invention, the process is carried out in such a way that the Lewis acid is used in an amount equal to or greater than 0.25 equivalents, per mol compound of formula (II). Preferably this amount is equal to or greater than 0.5 equivalents, more preferably equal to or greater than 0,75 equivalents, even more preferably equal to or greater than 1 equivalents, and most preferably equal to or greater than 1,1 equivalents per mol compound of formula (II). Generally, the Lewis acid is used in an amount equal to or smaller than 4 equivalents, per mol compound of formula (II). Preferably this amount is equal to or smaller than 3 equivalents, more preferably equal to or smaller than 2.5 equivalents, even more preferably equal to or smaller than 2 equivalents, and most preferably equal to or smaller than 1.5 equivalents per mol compound of formula (II). Preferably, the Lewis acid according to this embodiment is an aluminum or boron trialkyl compound, in particular trimethylaluminum.

In a second embodiment according to the present invention, the reaction is further carried out in the presence of an additional base which is different from the compound according to formula (III). In one aspect of this embodiment, the process is carried out in such a way that the Lewis acid is generally used in an amount equal to or greater than 0.25 equivalents, per mol ester of formula (II). Preferably this amount is equal to or greater than 0.5 equivalents, more preferably equal to or greater than 1.0 equivalents, even more preferably equal to or greater than 1.5 equivalents, and most preferably equal to or greater than 3 equivalents per mol ester of formula (II). Generally, the Lewis acid is used in an amount equal to or smaller than 8 equivalents, per mol ester of formula (II). Preferably this amount is equal to or smaller than 7 equivalents, more preferably equal to or smaller than 6 equivalents, even more preferably equal to or smaller than 5 equivalents, and most preferably equal to or smaller than 4 equivalents per mol ester of formula (II). In one aspect of this embodiment, the additional base is generally used in an amount equal to or greater than 0.05 equivalents, per mol Lewis acid. Preferably this amount is equal to or greater than 0.1 equivalents, more preferably equal to or greater than 0.15 equivalents, even more preferably equal to or greater than 0.2 equivalents, and most preferably equal to or greater than 0.25 equivalents per mol Lewis acid. Generally, the additional base is used in an amount equal to or smaller than 2 equivalents, per mol Lewis acid. Preferably this amount is equal to or smaller than 1.9 equivalents, more preferably equal to or smaller than 1.8 equivalents, even more preferably equal to or smaller than 1.7 equivalents, and most preferably equal to or smaller than 1.5 equivalents per mol Lewis acid. In one aspect of the second embodiment, the preferred Lewis acid is an aluminum or boron trihalide, in particular aluminum trichloride.

The term "additional base" preferably intends to denote a nucleophilic base. In another aspect, the term "additional base" intends to denote a non-nucleophilic base.

For the purpose of the present invention, the term "nucleophilic base" denotes a base which is capable to function as a nucleophile. Preferred nucleophilic bases are primary, secondary and tertiary amines, such as diethylamine, triethylamine, methylpiperidine and N-methylmorpholine, wherein triethylamine is the preferred base.

For the purpose of the present invention, the term "non-nucleophilic base" denotes a base which is at the same time a poor nucleophile.

Examples of suitable non-nucleophilic bases include sterically hindered alcoholates, such as potassium tert-butoxide (KOtBu), sodium tert-butoxide (NaOtBu) ; amines such as DBU (1,8-diazabicyclo[5.4.0]undec-7-ene), DBN (1,5-Diazabicyclo[4.3.0]non-5-ene), TMG (tetramethylguanidine), TBD (triazabicyclodecene) ; lithium compounds such as lithium diisopropylamide (LDA), tert-Butyllithium (tBuLi), lithium tetramethylpiperidide (Li-TMP) ; silicium compounds such as Sodium hexamethyldisilazane (Na-HMDS), Potassium hexamethyldisilazane (K-HMDS). Aluminium compounds such trimethyl aluminium. From among those, potassium tert-butoxide, trimethyl aluminium LDA, and DBU are more preferred. Most preferred base is potassium tert-butoxide.

If desired, the base may be an environmental friendly base which is for example prepared via a ring opening reaction of β or γ-lactones.

It has been found, in the first embodiment, that controlling the amount of the Lewis acid in the reaction medium improves the efficiency of the process and the yield and purity of the desired carboxamide of formula (I). In the second embodiment, controlling the amount of the Lewis acid and base in the reaction medium has been found to improve the efficiency of the process and the yield and purity of the desired carboxamide of formula (I). According to the second embodiment, the term "additional base" intends to denote a base that is not identical with the compound of formula (III). In a third embodiment, the compound of formula (III) is used in an excess of equal to or more than 1.2 molar equivalents, more preferably of equal to or more than 1.3, and even more preferably of equal to or more than 1.4 molar equivalents molar equivalents, with respect of the compound of formula (II), making the use of an additional base unnecessary. In another aspect of the invention, the addition of a base which is not identical with the compound of formula (III) can be advantageous even if the compound of formula (III) is used in an excess of more than 1.2 mol equivalents with respect to the amount of compund of formula (II).

The invention will be further described in more detail and the definitions and preferences described below for the compounds including starting compounds and target compounds and process conditions related to the process according to the invention equally apply to the first, second and third embodiment, indicated above and further embodiments, described below, of the process according to the invention.

The term "organic residue" is intended to denote in particular linear or branched alkyl or alkylene groups which may contain hetero atoms, such as in particular boron, silicon, nitrogen, oxygen or sulphur atoms and halogen atoms, cycloalkyl groups, heterocycles and aromatic systems. The organic residue may contain double or triple bonds and functional groups.

The organic residue comprises at least 1 carbon atom. It often comprises at least 2 carbon atoms. It preferably comprises at least 3 carbon atoms. More particularly preferably, it comprises at least 5 carbon atoms.

The organic residue generally comprises at most 100 carbon atoms. It often comprises at most 50 carbon atoms. It preferably comprises at most 40 carbon atoms. More particularly preferably, it comprises at most 30 carbon atoms.

R1 is typically selected from the group consisting of H, linear or branched alkyl or alkylene groups, cycloalkyl or cycloalkylene groups, heterocycles and aromatic systems, optionally containing heteroatoms, double bonds, triple bonds, functional groups and mixtures thereof. In a preferred aspect, R1 is selected from the group consisting of H, linear or branched alkyl or alkylene groups, cycloalkyl or cycloalkylene groups, heterocycles and aromatic systems, optionally containing heteroatoms, double bonds, triple bonds, functional groups and mixtures thereof ; preferably from H, C₁-C₈-alkyl, C₁-C₈-haloalkyl C₃-C₈-cycloalkyl, C₁-C₄ alkoxy-C₁-C₄ alkyl, C₃-C₈ cycloalkoxy-C₁-C₄ alkyl, C₂-C₈ alkenyl, and benzyl optionally substituted by 1, 2 or 3 substituents R^{Y1} independently of one another selected from C₁-C₄ alkyl, C₁-C₄ alkoxy and nitro ; more preferably from H, C₁-C₄-alkyl, C₁-C₄-haloalkyl, and benzyl; most preferably from methyl, ethyl, trifluoroethyl, pentafluoropropyl, hexafluoro-isopropyl, n-propyl, and isopropyl ; most preferably ethyl.

R2 is usually selected from the group consisting of H, linear or branched alkyl or alkylene groups, cycloalkyl or cycloalkylene groups, heterocycles and aromatic systems, optionally containing heteroatoms, double bonds, triple bonds, functional groups and mixtures thereof. In a preferred aspect, R2 is selected from the group consisting of H, linear or branched alkyl or alkylene groups, cycloalkyl or cycloalkylene groups, heterocycles and aromatic systems, optionally containing heteroatoms, double bonds, triple bonds, functional groups and mixtures thereof ; preferably from H, C₁-C₄ alkyl, benzyl and phenyl, where benzyl and phenyl may be optionally substituted by 1, 2 or 3 substituents R^{Y2} independently of one another selected from halogen, nitrile, nitro, C₁-C₄-alkyl, C₁-C₄-haloalkyl, C₁-C₄-alkoxy and C₁-C₄-haloalkoxy ; more preferably from H and C₁-C₄-alkyl ; most preferably methyl.

The term "aliphatic" intends to denote an acyclic or cyclic, saturated or unsaturated carbon compound, for example an alkyl group, cycloalkyl, alkylene, a halogenated alkyl group. A cyclic aliphatic, also denoted alicyclic, group has a carbocyclic ring structure which may be saturated or unsaturated, but may not be a benzenoid or other aromatic system. The term "aliphatic" comprises, for example, the term "alkyl group", "cycloalkyl group", "alkylene group" or "cycloalkylene group, or "halogenated alkyl group".

The term "heteroaliphatic" intends to denote an aliphatic group as defined above further comprising heteroatoms. A cyclic heteroaliphatic, also denoted heterocyclic, group has a carbocyclic ring structure further comprising one or more heteroatoms which may be saturated or unsaturated, but may not be an aromatic system. The term "heteroaliphatic" comprises, for example, the term "heterocycle".

The term "aromatic" typically intends to denote a carboyclic, cyclically conjugated molecular entity with a stability (due to delocalization) significantly greater than that of a hypothetical localized structure.

The term "heteroaromatic" typically intends to denote a heterocyclic, cyclically conjugated molecular entity with a stability (due to delocalization) significantly greater than that of a hypothetical localized structure. The term "alkyl group" is intended to denote in particular a linear or branched alkyl substituent comprising from 1 to 20 carbon atoms, preferably 1, 2, 3, 4, 5, 6, 7, 8, 9 or 10 carbon atoms. Specific examples of such substituents are methyl, ethyl, n-propyl, isopropyl, n-butyl, isobutyl, tert-butyl, n-pentyl, isopentyl, n-hexyl, 2-hexyl, n-heptyl, n-octyl and benzyl.

The term "cycloalkyl group" is intended to denote in particular a substituent comprising at least one saturated carbocycle containing 3 to 10 carbon atoms, preferably 5, 6 or 7 carbon atoms. Specific examples of such substituents are cyclopropyl, cyclobutyl, cyclopentyl, cyclohexyl and cycloheptyl.

The term "alkylene group" or "cycloalkylene group" is intended to denote in particular the divalent radicals derived from the alkyl or cycloalkyl groups as defined above.

When the organic residue contains one or optionally more double bonds, it is often chosen from an alkenyl or cycloalkenyl group comprising from 2 to 20 carbon atoms, preferably 2, 3, 4, 5, 6, 7, 8, 9 or 10 carbon atoms. Specific examples of such groups are vinyl, 1-allyl, 2-allyl, n-but-2-enyl, isobutenyl, 1,3-butadienyl, cyclopentenyl, cyclohexenyl and styryl.

When the organic residue contains one or optionally more triple bonds, it is often chosen from an alkinyl group comprising from 2 to 20 carbon atoms, preferably 2, 3, 4, 5, 6, 7, 8, 9 or 10 carbon atoms. Specific examples of such groups are ethinyl, 1-propinyl, 2-propinyl, n-but-2-inyl and 2-phenylethinyl.

When the organic residue contains one or optionally more aromatic systems, it is often an aryl group comprising from 6 to 24 carbon atoms, preferably from 6 to 12 carbon atoms. Specific examples of such groups are phenyl, 1-tolyl, 2-tolyl, 3-tolyl, xylyl, 1-naphthyl and 2-naphthyl.

The term "heterocycle" is intended to denote in particular a cyclic system comprising at least one saturated or unsaturated ring made up of 3, 4, 5, 6, 7 or 8 atoms, at least one of which is a hetero atom. The hetero atom is often chosen from B, N, O, Si, P and S. It is more often chosen from N, O and S.

Specific examples of such heterocycles are aziridine, azetidine, pyrrolidine, piperidine, morpholine, 1,2,3,4-tetrahydroquinoline, 1,2,3,4-tetrahydroisoquinoline, perhydroquinoline, perhydroisoquinoline, isoxazolidine, pyrazoline, imidazoline, thiazoline, tetrahydrofuran, tetrahydrothiophene, pyran, tetrahydropyran and dioxane.

The organic residues as defined above may be unsubstituted or substituted with functional groups. The term "functional group" is intended to denote in particular a substituent comprising or consisting of a hetero atom. The hetero atom is often chosen from B, N, O, Al, Si, P, S, Sn, As and Se and the halogens. It is more often chosen from N, O, S and P, in particular N, O and S.

The functional group generally comprises 1, 2, 3, 4, 5 or 6 atoms.

By way of functional groups, mention may, for example, be made of halogens, a hydroxyl group, an alkoxy group, a mercapto group, an amino group, a nitro group, a carbonyl group, an acyl group, an optionally esterified carboxyl group, a carboxamide group, a urea group, a urethane group and the thiol derivatives of the abovementioned groups containing a carbonyl group, phosphine, phosphonate or phosphate groups, a sulphoxide group, a sulphone group and a sulphonate group.

The term "halogenated alkyl group" is intended to denote in particular an alkyl group comprising from 1 to 20 carbon atoms and at least one halogen, preferably 1, 2, 3, 4, 5, 6, 7, 8, 9 or 10 carbon atoms and at least one halogen. Suitable halogenated alkyl groups are selected for example from chlorinated alkyl groups such as chloromethyl, dichloromethyl, trichloromethyl, 1-chloroethyl or 2,2,2-trichloroethyl fluorinated alkyl groups such as fluoromethyl, difluoromethyl, trifluoromethyl, 1 -fluoroethyl, 2-fluoroethyl, 2,2-difluoroethyl, 2,2,2-trifluoroethyl or pentafluoroethyl, chlorofluorinated alkyl groups such as chlorofluoromethyl, dichlorofluoromethyl, chlorodifluoromethyl, 2-chloro-2-fluoroethyl, 2-chloro-2,2-difluoroethyl or 2,2- dichloro-2-fluoroethyl, brominated alkyl groups such as bromomethyl and 1-bromoethyl.

In a preferred embodiment of the process according to the invention, R1 is C₁-C₈-alkyl, C₁-C₈-haloalkyl, C₃-C₈-cycloalkyl, C₁-C₄-alkoxy-C₁-C₄-alkyl, C₃-C₈-cycloalkoxy-C₁-C₄-alkyl, C₂-C₈-alkenyl or is benzyl which is optionally substituted by 1,2 or 3 substituents R^{Y1} independently of one another selected from the group consisting of C₁-C₄-alkyl, C₁-C₄-alkoxy and nitro; and

R2 is hydrogen, C₁-C₄-alkyl, benzyl or phenyl, where the two last-mentioned substituents may be unsubstituted or optionally substituted by 1,2 or 3 substituents R^{Y2} independently of one another selected from the group consisting of halogen, nitrile, nitro, C₁-C₄-alkyl, C₁-C₄-haloalkyl, C₁-C₄-alkoxy and C₁-C₄-haloalkoxy; and

R3 is an alkyl group or a halogenated alkyl group. R₄ is hydrogen, C₁-C₈-alkyl, benzyl or phenyl.

The terms, used in the definition of the variables, for organic groups, such as, for example, the term "halogen", are collective terms representing the individual members of these groups of organic moieties.

The prefix Cₓ-C_{y} denotes the number of possible carbon atoms in the case in question. C₁-C₄-Alkyl includes, for example, methyl, ethyl, propyl, 1-methylethyl, butyl, 1-methylpropyl, 2-methylpropyl or 1,1-dimethylethyl.

The term "halogen" denotes in each case fluorine, bromine, chlorine or iodine, especially fluorine, chlorine or bromine. This also applies, correspondingly, to halogen in combination with other meanings, such as haloalkyl or haloalkoxy.

The term "alkoxy" is, for example, methoxy, ethoxy, propoxy, i-propoxy, n-butoxy, isobutoxy, sec-butoxy and tert-butoxy ; preferably methoxy and ethoxy.

Haloalkoxy is, for example, fluoromethoxy, difluoromethoxy, trifluoromethoxy, 2,2,2-trifluoroethoxy, 1,1,2,2-tetrafluoroethoxy, 2-fluoroethoxy, 2-chloroethoxy, 2,2-difluoroethoxy and 2,2,2-trichloroethoxy ; preferably difluoromethoxy, 2-chloroethoxy and trifluoromethoxy. Alkylthio is, for example, methylthio, ethylthio, propylthio, isopropylthio, n-butylthio, isobutylthio, sec-butylthio or tert-butylthio, preferably methylthio and ethylthio.

The term "C₁-C₄-alkoxy-C₁-C₄-alkyl", as used herein, describes C₁-C₄-alkyl radicals where one carbon atom is attached to a C₁-C₄-alkoxy radical. Examples of these are CH₂-OCH₃, CH₂-OC₂H₅, n-propoxymethyl, CH₂-OCH(CH₃)₂, n-butoxymethyl, (1-methylpropoxy)methyl, (2-methylpropoxy)methyl, CH₂-OC(CH₃)₃, 2-(methoxy)ethyl, 2-(ethoxy)ethyl, 2-(n-propoxy)ethyl, 2-(1-methylethoxy)ethyl, 2-(n-butoxy)ethyl, 2-(1-methylpropoxy)ethyl, 2-(2-methylpropoxy)ethyl, 2-(1,1-dimethylethoxy)ethyl, 2-(methoxy)propyl, 2-(ethoxy)propyl, 2-(n-propoxy)propyl, 2-(1-methylethoxy)propyl, 2-(n-butoxy)propyl, 2-(1-methylpropoxy)propyl, 2-(2-methylpropoxy)propyl, 2-(1,1-dimethylethoxy)propyl, 3-(methoxy)propyl, 3-(ethoxy)propyl, 3-(n-propoxy)propyl, 3-(1-methylethoxy)propyl, 3-(n-butoxy)propyl, 3-(1-methylpropoxy)propyl, 3-(2-methylpropoxy)propyl, 3-(1,1-dimethylethoxy)propyl, 2-(methoxy) butyl, 2-(ethoxy)butyl, 2-(n-propoxy)butyl, 2-(1-methylethoxy)butyl, 2-(n-butoxy)butyl, 2-(1-methylpropoxy)butyl, 2-(2-methylpropoxy)butyl, 2-(1,1-dimethylethoxy)butyl, 3-(methoxy)butyl, 3-(ethoxy)butyl, 3-(n-propoxy)butyl, 3-(1-methylethoxy)butyl, 3-(n-butoxy)butyl, 3-(1-methylpropoxy)butyl, 3-(2-methylpropoxy)butyl, 3-(1,1-dimethylethoxy)butyl, 4-(methoxy)butyl, 4-(ethoxy)butyl, 4-(n-propoxy)butyl, 4-(1-methylethoxy)butyl, 4-(n-butoxy)butyl, 4-(1-methylpropoxy)butyl, 4-(2-methylpropoxy) butyl, 4- 1,1-dimethylethoxy)butyl.

The term "C₂-C₈-alkenyl", as used herein, describes straight-chain and branched unsaturated hydrocarbon radicals having 2 to 8 carbon atoms and at least one carbon-carbon double bond, such as, for example, ethenyl, 1-propenyl, 2-propenyl, 1-methylethenyl, 1-butenyl, 2-butenyl, 3-butenyl, 1-methyl-1-propenyl, 2-methyl-1-propenyl, 1-methyl-2-propenyl, 2-methyl-2-propenyl, 1-pentenyl, 2-pentenyl, 3-pentenyl, 4-pentenyl, 1-methyl-1-butenyl, 2-methyl-1-butenyl, 3-methyl-1-butenyl, 1-methyl-2-butenyl, 2-methyl-2-butenyl, 3-methyl-2-butenyl, 1-methyl-3-butenyl, 2-methyl-3-butenyl, 3-methyl-3-butenyl, 1,1-dimethyl-2-propenyl, 1,2-dimethyl-1-propenyl, 1,2-dimethyl-2-propenyl, 1-ethyl-1-propenyl, 1-ethyl-2-propenyl, 1-hexenyl, 2-hexenyl, 3-hexenyl, 4-hexenyl, 5-hexenyl, 1-methyl-1-pentenyl, 2-methyl-1-pentenyl, 3-methyl-1-pentenyl, 4-methyl-1-pentenyl, 1-methyl-2-pentenyl, 2-methyl-2-pentenyl, 3-methyl-2-pentenyl, 4-methyl-2-pentenyl, 1-methyl-3-pentenyl, 2-methyl-3-pentenyl, 3-methyl-3-pentenyl, 4-methyl-3-pentenyl, 1-methyl-4-pentenyl, 2-methyl-4-pentenyl, 3-methyl-4-pentenyl, 4-methyl-4-pentenyl, 1,1-dimethyl-2-butenyl, 1,1-dimethyl-3-butenyl, 1,2-dimethyl-1-butenyl, 1,2-dimethyl-2-butenyl, 1,2-dimethyl-3-butenyl, 1,3-dimethyl-1-butenyl, 1,3-dimethyl-2-butenyl, 1,3-dimethyl-3-butenyl, 2,2-dimethyl-3-butenyl, 2,3-dimethyl-1-butenyl, 2,3-dimethyl-2-butenyl, 2,3-dimethyl-3-butenyl, 3,3-dimethyl-1-butenyl, 3,3-dimethyl-2-butenyl, 1-ethyl-1-butenyl, 1-ethyl-2-butenyl, 1-ethyl-3-butenyl, 2-ethyl-1-butenyl, 2-ethyl-2-butenyl, 2-ethyl-3-butenyl, 1,1,2-trimethyl-2-propenyl, 1-ethyl-1-methyl-2-propenyl, 1-ethyl-2-methyl-1-propenyl and 1-ethyl-2-methyl-2-propenyl, 1-heptenyl, 2-heptenyl, 1-octenyl or 2-octenyl.

In a preferred embodiment of the process according to the invention, X is oxygen, R1 is C₁-C₄-alkyl, C₁-C₄-haloalkyl or benzyl, in particular methyl, ethyl, trifluoroethyl, pentafluoropropyl, hexafluoro-iso-propyl,n-propyl or isopropyl; R1 is especially ethyl; and
R2 is H or C₁-C₄-alkyl ; R2 is especially methyl; R3 is selected from a group consisting of fluoromethyl, difluoromethyl, trifluoromethyl, chlorofluoromethyl, dichlorofluoromethyl, and chlorodifluoromethyl ; R3 is especially difluoromethyl. R4 is selected from the group consisting of hydrogen and C₁-C₄-alkyl ; R4 is especially hydrogen ; Q is Q1 or Q37.

In one embodiment, Q is Q1 and R⁶ is a hydrogen, C₁₋₁₂ alkyl, C₂₋₁₂ alkenyl or C₂₋₁₂ alkynyl group, which may be substituted by 1 to 6 substituents, each substituent independently selected from halogen, cyano, C₁₋₄ alkoxy, C₁₋₄ thioalkyl, COO-C₁₋₄ alkyl, =N-OH, =N-O-(C₁₋₄ alkyl), C₃₋₈ cycloalkyl, which may itself be substituted by 1 to 3 substituents, each independently selected from C₁₋₄ alkyl, halogen, C₁₋₄ alkoxy and C₁₋₄ haloalkoxy, and C₄₋₈ cycloalkenyl, which may itself be substituted by 1 to 3 substituents, each independently selected from C₁₋₄ alkyl, halogen, C₁₋₄ alkoxy and C₁₋₄ haloalkoxy, R⁶ is especially a hydrogen ; R⁷, R⁸, R⁹ and R¹⁰ are each, independently, hydrogen, halogen, C₁₋₄ alkyl, C₁₋₄ haloalkyl, C₁₋₄ alkoxy, C₁₋₄ haloalkoxy, R⁷, R⁸, R⁹ and R¹⁰ are especially each, independently, hydrogen and halogen, said halogen is especially chlorine or fluorine.

In another embodiment, Q is Q1 and R⁶ is phenyl, which may be substituted by 1 to 3 substituents, each independently selected from halogen, cyano, nitro, C₁₋₄ alkyl, C₁₋₄ haloalkyl, C₁₋₄ alkoxy, C₁₋₄ alkylthio, C₁₋₄ haloalkoxy, C₁₋₄ haloalkylthio, C(H)=N-OH, C(H)=N-O(C₁₋₆ alkyl), C(C₁₋₆ alkyl)=N-OH, C(C₁₋₆ alkyl)=N-O-(C₁₋₆ alkyl), (Z)C≡CR, (Z)ₙCR²⁸ =CR²⁶ R²⁷, phenyl, which may itself be substituted by 1 to 3 substituents, each independently selected from halogen, cyano, nitro, C₁₋₄ alkyl, C₁₋₄ haloalkyl, C₁₋₄ alkoxy, C₁₋₄ haloalkoxy, C₁₋₄ haloalkylthio, C(H)=N-OH, C(H)=N-O(C₁₋₆ alkyl), C(C₁₋₆ alkyl)=N-OH and C(C₁₋₆ alkyl)=N-O-(C₁₋₆ alkyl), and thienyl, which may itself be substituted by 1 to 3 substituents, each independently selected from halogen, cyano, nitro, C₁₋₄ alkyl, C₁₋₄ haloalkyl, C₁₋₄ alkoxy, C₁₋₄ haloalkoxy, C₁₋₄ haloalkylthio, C(H)=N-OH, C(H)=N-O(C₁₋₆ alkyl), C(C₁₋₆ alkyl)=N-OH and C(C₁₋₆ alkyl)=N-O-(C₁₋₆ alkyl) ; R⁶ is especially phenyl, which is substituted in the para-position by halogen, wherein said phenyl may be further substituted by 1 to 2 substituents, each independently selected from halogen, C₁₋₄ alkyl and C₁₋₄ haloalkyl; R⁷, R⁸, R⁹ and R¹⁰ are especially each, independently, hydrogen and halogen, said halogen is especially chlorine or fluorine.

In another embodiment, Q is Q1 and R⁶ is a C₃₋₈ cycloalkyl, C₄₋₈ cycloalkenyl or C₅₋₈ cycloalkadienyl group, which may be substituted by 1 to 3 substituents, each independently selected from halogen, C₁₋₄alkyl, C₁₋₄ haloalkyl, C₁₋₄ alkoxy, C₁₋₄ haloalkoxy, C₁₋₄ thioalkyl, C₃₋₆ cycloalkyl, which may itself be substituted by 1 to 3 substituents, each independently selected from C₁₋₄ alkyl, halogen, C₁₋₄ alkoxy and C₁₋₄ haloalkoxy, and phenyl, which may itself be substituted by 1 to 5 independently selected halogen atoms ; R⁶ is especially a C₃₋₈ cycloalkyl, which may be substituted by 1 to 3 substituents, each independently selected from halogen, C₁₋₄alkyl, C₁₋₄ haloalkyl.

In another embodiment, Q is Q37 and R¹³, R¹⁴, R¹⁵ and R¹⁶ are each, independently, hydrogen, halogen, C₁₋₄ alkyl, C(O)CH₃, C₁₋₄ haloalkyl, C₁₋₄ alkoxy ; in particular R¹⁴, R¹⁵ and R¹⁶ are each, independently, hydrogen, methyl, methoxy or C(O)CH₃ ; R¹³, R¹⁴, R¹⁵ and R¹⁶ are especially each, independently, hydrogen or methyl and W is a single bound ; and Y is O or (CR¹⁹R²⁰)(CR²¹R²²)ₘ₁(CR²³R²⁴)ₙ1 ; preferably Y is (CR¹⁹R²⁰)(CR²¹R²²)ₘ₁(CR²³R²⁴)ₙ₁, more preferably Y is (CR¹⁹R²⁰)(CR²¹R²²)ₘ₁ and R¹⁹, R²⁰, R²¹, R²², R²³ and R²⁴ are each independently 1 hydrogen, halogen, C₁₋₄ alkoxy, C₁₋₆ alkyl, which may be substituted by 1 to 3 substituents selected from halogen, hydroxy, =O, C₁₋₄ alkoxy, O-C(O)-C₁₋₄ alkyl, phenyl, naphthyl, anthracyl, fluorenyl, indanyl or a 3-7 membered carbocyclic ring (which itself may be substituted by 1 to 3 methyl groups), C₁₋₆ alkenyl, which may be substituted by 1 to 3 substituents selected from halogen, hydroxy, =O, C₁₋₄ alkoxy, O-C(O)-C₁₋₄ alkyl, phenyl, naphthyl, anthracyl, fluorenyl, indanyl or a 3-7 membered carbocyclic ring (which itself may be substituted by 1 to 3 methyl groups), or a 3- 7 membered carbocyclic ring, which may contain 1 heteroatom selected from nitrogen and oxygen, and wherein the 3-7 membered carbocyclic ring may be substituted by 1 to 3 methyl groups ; or R¹⁹, R²⁰ together with the carbon atom to which they are attached form a carbonyl-group, a 3-5 membered carbocyclic ring, which may be substituted by 1 to 3 methyl groups, C₁₋₆ alkylidene, which may be substituted by 1 to 3 methyl groups, or C₃₋₆ cycloalkylidene, which may be substituted by 1 to 3 methyl groups ; especially R¹⁹ and R²⁰ together with the carbon atom to which they are attached form a 3-membered or 5- membered carbocyclic ring ; preferably R²¹, R²², R²³ and R²⁴ are each independently hydrogen or CH₃.

In a first specific preferred embodiment, Q is a group of formula Q39 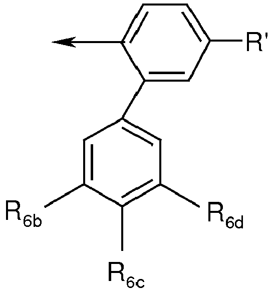 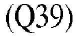 wherein R', R_{6b}, R_{6c} and R_{6d} are each, independently, hydrogen or halogen, said halogen is especially chlorine or fluorine.

In a second specific preferred embodiment, Q is a group of formula Q40

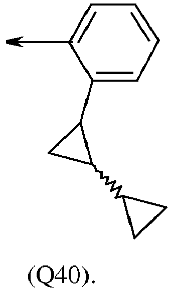

In a third specific preferred embodiment, Q is a group of formula Q41

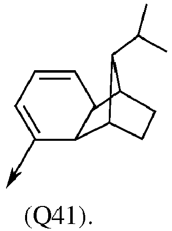

In a particularly preferred aspect of the process of the present invention, an amine of formula (III): QNHR4 (III) wherein Q is selected from a group consisting of Q35 to Q41, is reacted with ethyl 1-methyl-3-difluoromethyl-pyrazole-4-carboxylate (DFMMP).

Amines of the formula (III) are either known, for example, from EP 1490342, EP 0824099, EP 1480955 (B1), WO 2004/035589, WO 2007/031323, or they can be prepared according to generally known methods.

In one particular embodiment of the present invention, the process according to the present invention is comprised in a process for the manufacture of pharmaceutically active or agrochemically active compounds or their precursors. Such a process can also comprise further processes or process steps.

The formation of the compound of formula (II) can be carried out, for example, analogously to the reaction described in the patent applications EP - 10170633.1 and EP - 10173899.5. The respective content of said patent applications is incorporated by reference into the present patent application.

In the process according to the invention, the reaction is generally carried out in an inert solvent. Examples of suitable inert solvents include hydrocarbons such as benzene, toluene, xylene or cyclohexane; halogenated hydrocarbons such as dichloromethane, trichloromethane or tetrachloromethane ; halogenated aromatic hydrocarbons such as chlorobenzene, straight chain or cyclic ethers such as diethyl ether, ethylene glycol dimethyl ether, diethylene glycol dimethyl ether, tetrahydrofuran or dioxane, nitriles such as acetonitrile or propionitrile, amides such as N,N-dimethylformamide, diethylformamide or N-methylpyrrolidinone ; these inert solvents can be used alone or in combination as a mixture.

In a preferred specific embodiment, the solvent is selected from the group consisting of halogenated hydrocarbons such as dichloromethane, trichloromethane or tetrachloromethane; and straight chain or cyclic ethers such as diethyl ether, ethylene glycol dimethyl ether, diethylene glycol dimethyl ether, tetrahydrofuran or dioxane. In a further specific embodiment, the solvent is different than chlorobenzene, more particularly different than a halogenated aromatic hydrocarbon.

The reaction is preferably carried out in a straight chain or cyclic ether, in particular in a cyclic ether and particularly preferable in tetrahydrofuran (THF) or dioxane.

Generally, an excess of the compound of formula (II) or (III) can also be employed as solvent.

In a preferred aspect of the process of the present invention, the solvent is substantially free of water.

For the purpose of the present invention, the term "solvent substantially free of water" denotes in particular that the content of water in the solvent is equal to or lower than 3100 mg/kg of water, preferably equal to or lower than 500 mg/kg of water, more preferably equal to or lower than 400 mg/kg of water, most preferably equal to or lower than 50 mg/kg of water. The solvent can be completely anhydrous. However, the solvent substantially free of water generally contains at least 5 mg/kg of water, often at least 25 mg/kg of water. Solvents which are substantially free of water allow shorter residence time and/or lower temperatures thereby leading to a more economical and environmental beneficial process.

If appropriate, the solvent is used usually in an amount of from 50 to 99 by weight, preferably from 60 to 99 % by weight, more preferably from 75 to 99 % by weight of the solvent relative to the total weight of the reaction medium.

The process according to the invention is, if appropriate, carried out in the presence of a suitable phase transfer catalyst such as for example a crown ether. This allows to increase the yield and to reduce the reaction time.

If desired, the solvent can be chosen on account of respective pKa of base and reagents.

In the process according to the invention, the temperature of the reaction is generally at least 0°C. The temperature of the reaction is often at least 15°C. Preferably, this temperature is at least 25°C. The temperature of the reaction is generally at most the boiling temperature of the solvent. Typically, the temperature of the reaction is equal to or lower than 120°C, particularly equal to or lower than 110°C, more particularly equal to or lower than 100°C, most particularly equal to or lower than 90°C, a temperature equal to or lower than 80°C being especially suitable. A temperature from 15 to 100°C is suitable, a temperature from 15 to 90°C is particularly preferred, a temperature from 15 to 80°C is most particularly preferred.

Should the disclosure of any patents, patent applications, and publications which are incorporated herein by reference conflict with the description of the present application to the extent that it may render a term unclear, the present description shall take precedence.

The following examples are intended to further explain the invention without limiting it.

In these examples and throughout this specification the abbreviations employed are defined as follows : DFMMP is ethyl 1-methyl-3-difluoromethyl-pyrazole-4-carboxylate. 2-(3,4,5-trifluorophenyl)aniline can be prepared, for example, according to US2011/301356. DFMMP, can, for example, be prepared according to WO2012/25469. 2-(bi(cyclopropan)-2-yl)benzenamine can, for example, be prepared according to EP2014642.

### Example 1 : Synthesis of Fluxapyroxad^{®}

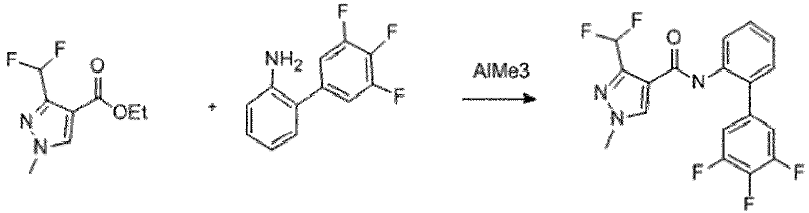

A mixture of 1.25 g (6.11 mmol, 1.0 eq) DFMMP and 1.50 g (6.73 mmol, 1.10 eq) 2-(3,4,5-trifluorophenyl)aniline was placed in a flame-dried flask without addition of solvent. With stirring, 4.58 ml (9.17 mmol, 1.5 eq) a 2 M solution of trimethyl aluminium in toluene was added rapidly at a rate which led to boiling of the reaction mixture. The reaction was kept at 90°C for 3 h. The reaction mixture was allowed to cool to room temperature over night, poured on water and extracted with 200 ml ethyl acetate. The organic layer was washed with brine, dried over Na₂SO₄, and the solvent was removed in vacuum, giving 2.20 g crude product as a brown solid. The solid was washed thoroughly with ethyl acetate, yielding 1.70 g pure product as a light brown solid.

Evaporation of the mother liquor and column chromatography of the residue on silica (n-hexanes/EE 2:1) yields further 100 mg for a total of 1.80 g product (77 %).

HPLC/MS tᵣ: 12.6 min, [2M+Na]⁺ 785, [M+Na]⁺ 404, [M+H]⁺ 382; GC/MS tᵣ: 25.26 min, [M]⁺ 381; 159; ¹H-NMR (90 MHz, CDCl₃): δ (ppm) = 8.3-7.0 (m, 5H, Ar), 6.6 (t, 1H, CF₂H), 3.9 (s, 3H, CH₃

### Example 2 : Synthesis of Sedaxane^{®}

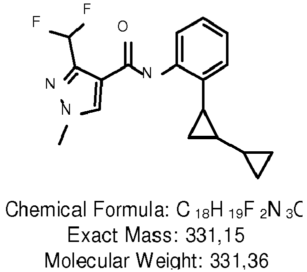

I To a solution of 2.93 g (14.3 mmol, 1.00 eq) DFMMP and 3.00 g (17.3 mmol, 1.20 eq) 2-(bi(cyclopropan)-2-yl)benzenamine in 20 mL dry dioxane, 10.0 ml (72.2 mmol, 5.40 eq) triethylamine was added, followed by 7.20 g (57.1 mmol, 4.00 eq) AlCl₃ which was added in one portion. After stirring the reaction mixture for 1 h, it was cooled with ice and quenched with water. The aqueous phase was extracted twice with ethyl acetate, the combined organic layers were washed with water and brine, dried over Na₂SO₄ and the solvent removed under reduced pressure. The crude product was purified by column chromatography (MTBE) to yield (4.1 g, 85 %) Sedaxane^{®}.

HPLC: tᵣ = 10.9 min + 11.4 min (*trans* +*cis* 62:38); HPLC/MS: tᵣ = 12.7 min [M+H]⁺ 332; tᵣ = 13.3 min [M+H]⁺ 332; NMR (500 MHz, CDCl₃) δ (ppm) = 8.46 (s, br, 0.35H), 8.08 (s, br, 0.65H), 8.26 (d, ³J = 8.2 Hz, 0.35H), 8.07 (d, ³J = 8.1 Hz, 0.65H), 7.98 (d, ³J = 6.3 Hz, 1H), 7.27-7.20 (m, 1H), 7.09-7.04 (m, 2H), 7.02-6.80 (m, 1H), 3.95 (s, 1H), 3.94 (s, 2H), 1.98-1.93 (m, 0.35H), 1.66-1.62 (m, 0.65H), 1.17-1.13 (m, 0.65H), 1.07-1.02 (m, 0.35H), 0.97-0.87 (m, 1H), 0.80-077 (m, 1H), 0.71-0.67 (m, 0.65H), 0.42-0.38 (m, 1.35H), 0.30-0.25 (m. 0.35H), 0.21-0.11 (m, 1.65H), 0.07-0.01 (m, 1H).

## Claims

1. A process for the manufacture of compounds of formula (I) 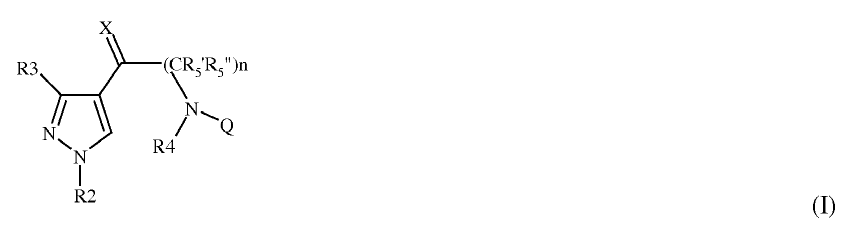 wherein
- R2 is H or an organic residue
- R3 is H, an alkyl group having from 1 to 12 carbon atoms, a halogenated alkyl group having from 1 to 12 carbon atoms, an aralkyl group, an aryl group, a halogen
- R4 is H, an alkyl group having from 1 to 12 carbon atoms, a halogenated alkyl group having from 1 to 12 carbon atoms, an aralkyl group, an aryl group, a halogen
- R5' and R5" are each independently selected from H, an alkyl group having from 1 to 12 carbon atoms, a halogenated alkyl group having from 1 to 12 carbon atoms, an aralkyl group, an aryl group, a halogen
- n = 0 to 10
- X is oxygen or sulfur
- Q is a group of any of formulae (Q1) to (Q38) herein below: 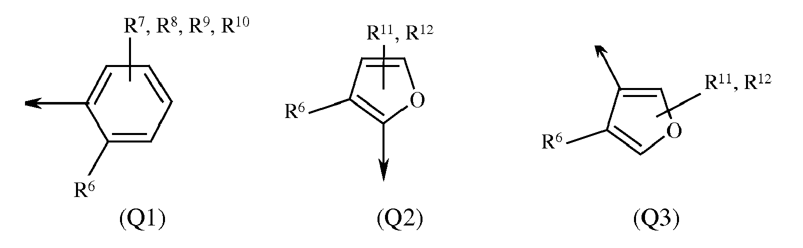 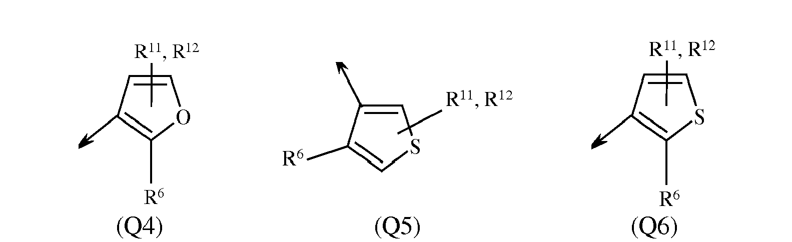 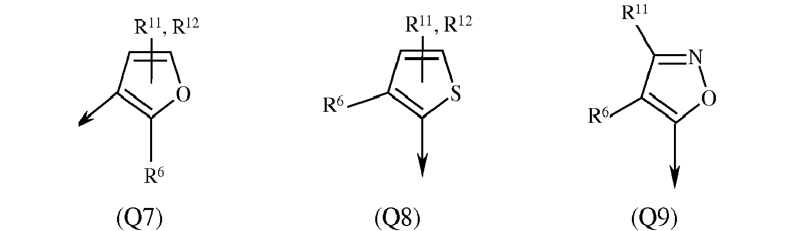 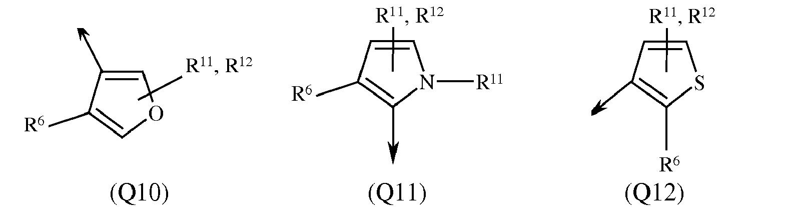 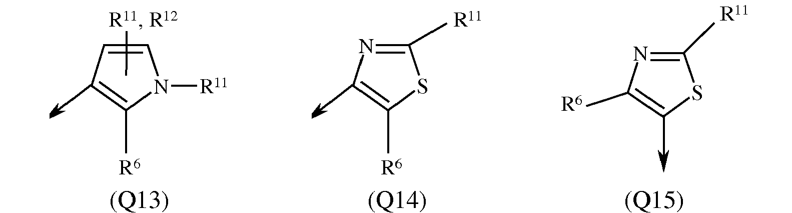 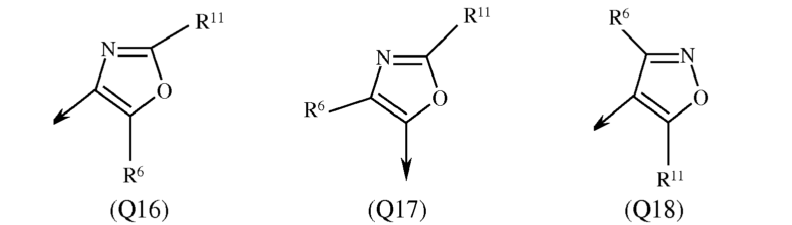 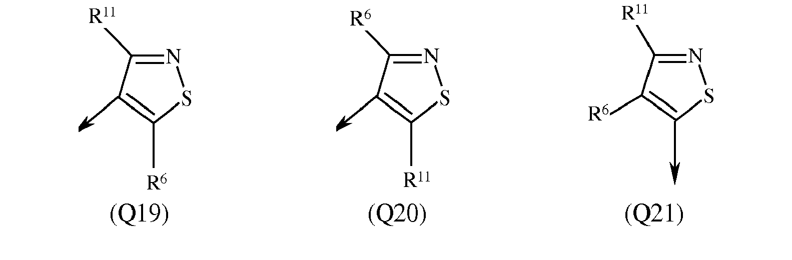 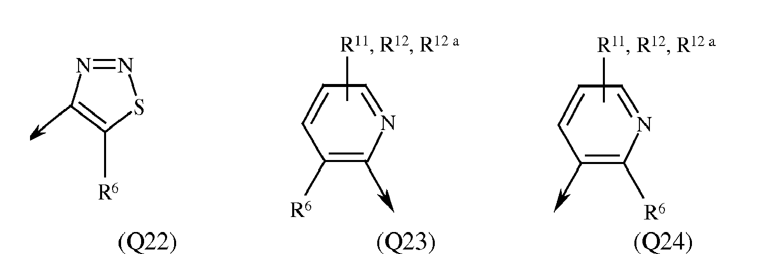 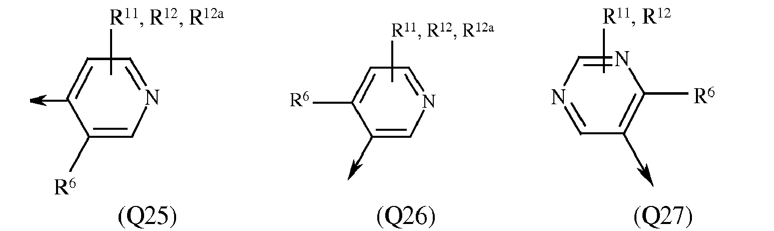 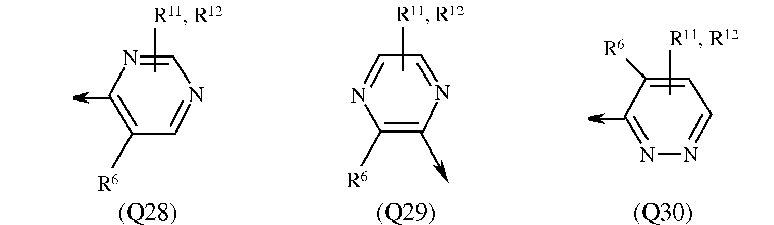 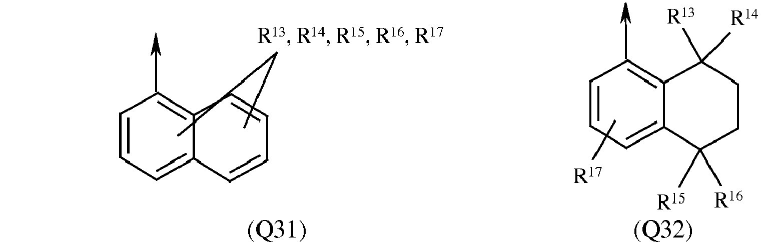 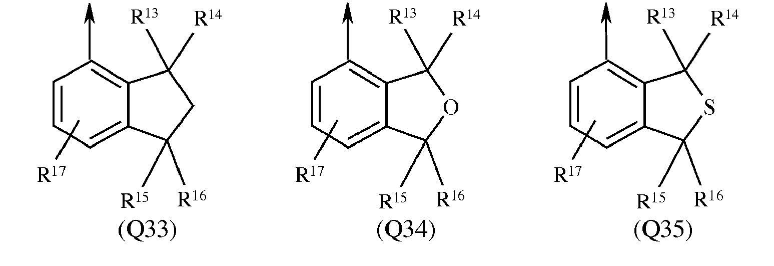 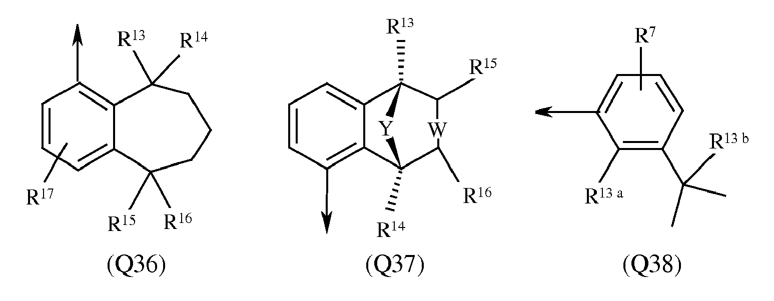
wherein R⁶ is a hydrogen, C₁₋₁₂ alkyl, C₂₋₁₂ alkenyl or C₂₋₁₂ alkynyl group, which may be substituted by 1 to 6 substituents, each substituent independently selected from halogen, cyano, C₁₋₄ alkoxy, C₁₋₄ thioalkyl, COO-C₁₋₄ alkyl, =N-OH, =N-O-(C₁₋₄ alkyl), C₃₋₈ cycloalkyl, which may itself be substituted by 1 to 3 substituents, each independently selected from C₁₋₄ alkyl, halogen, C₁₋₄ alkoxy and C₁₋₄ haloalkoxy, and C₄₋₈ cycloalkenyl, which may itself be substituted by 1 to 3 substituents, each independently selected from C₁₋₄ alkyl, halogen, C₁₋₄ alkoxy and C₁₋₄ haloalkoxy ;
or R⁶ is a C₃₋₈ cycloalkyl, C₄₋₈ cycloalkenyl or C₅₋₈ cycloalkadienyl group, which may be substituted by 1 to 3 substituents, each independently selected from halogen, C₁₋₄ alkyl, C₁₋₄ haloalkyl, C₁₋₄ alkoxy, C₁₋₄ haloalkoxy, C₁₋₄ thioalkyl, C₃₋₆ cycloalkyl, which may itself be substituted by 1 to 3 substituents, each independently selected from C₁₋₄ alkyl, halogen, C₁₋₄ alkoxy and C₁₋₄ haloalkoxy, and phenyl, which may itself be substituted by 1 to 5 independently selected halogen atoms ;
or R⁶ is a C₆₋₁₂ bicycloalkyl, C₆₋₁₂ bicycloalkenyl or C₆₋₁₂ bicycloalkadienyl group, which may be substituted by 1 to 3 substituents, each independently selected from halogen, C₁₋₄ alkyl and C₁₋₄ haloalkyl ;
or R⁶ is phenyl, which may be substituted by 1 to 3 substituents, each independently selected from halogen, cyano, nitro, C₁₋₄ alkyl, C₁₋₄ haloalkyl, C₁₋₄ alkoxy, C₁₋₄ alkylthio, C₁₋₄ haloalkoxy, C₁₋₄ haloalkylthio, C(H)=N-OH, C(H)=N-O(C₁₋₆ alkyl), C(C₁₋₆ alkyl)=N-OH, C(C₁₋₆ alkyl)=N-O-(C₁₋₆ alkyl), (Z)C≡CR, (Z)ₙCR²⁸ =CR²⁶ R²⁷, phenyl, which may itself be substituted by 1 to 3 substituents, each independently selected from halogen, cyano, nitro, C₁₋₄ alkyl, C₁₋₄ haloalkyl, C₁₋₄ alkoxy, C₁₋₄ haloalkoxy, C₁₋₄ haloalkylthio, C(H)=N-OH, C(H)=N-O(C₁₋₆ alkyl), C(C₁₋₆ alkyl)=N-OH and C(C₁₋₆ alkyl)=N-O-(C₁₋₆ alkyl), and thienyl, which may itself be substituted by 1 to 3 substituents, each independently selected from halogen, cyano, nitro, C₁₋₄ alkyl, C₁₋₄ haloalkyl, C₁₋₄ alkoxy, C₁₋₄ haloalkoxy, C₁₋₄ haloalkylthio, C(H)=N-OH, C(H)=N-O(C₁₋₆ alkyl), C(C₁₋₆ alkyl)=N-OH and C(C₁₋₆ alkyl)=N-O-(C₁₋₆ alkyl) ;
or R⁶ is a 5-6 membered heterocyclic ring, wherein the heterocyclic ring contains 1 to 3 heteroatoms, each heteroatom independently chosen from oxygen, sulphur and nitrogen, wherein the heterocyclic ring may be substituted 1 to 3 substituents, each independently selected from halogen, cyano, nitro, C₁₋₄ alkyl, C₁₋₄ haloalkyl, C₁₋₄ alkoxy, C₁₋₄ alkylthio, C₁₋₄ alkylthio, C₁₋₄ haloalkoxy, C(H)=N-O-(C₁₋₆ alkyl) and C(C₁₋₆ alkyl)=N-O-(C₁₋₆ alkyl), C₂₋₅ alkenyl, C₂₋₅ alkynyl, CHO, COOC₁-C₆ alkyl, C₁-C₄ alkoxy-C₁-C₄ alkyl, C₁-C₄ haloalkoxy-C₁-C₄ alkyl, (Z)_{P}C≡CR, (Z)ₙCR²⁸ =CR²⁶ R²⁷, phenyl, which may itself be substituted by 1 to 3 substituents, each independently selected from halogen, cyano, nitro, C₁₋₄alkyl, C₁₋₄ haloalkyl, C₁₋₄ alkoxy, C₁₋₄ haloalkoxy, C₁₋₄ haloalkylthio, C(H)=N-OH, C(H)=N-O(C₁₋₆ alkyl), C(C₁₋₆ alkyl)=N-OH and C(C₁₋₆ alkyl)=N-O-(C₁₋₆ alkyl), and thienyl, which may itself be substituted by 1 to 3 substituents, each independently selected from halogen, cyano, nitro, C₁₋₄ alkyl, C₁₋₄ haloalkyl, C₁₋₄ alkoxy, C₁₋₄ haloalkoxy, C₁₋₄ haloalkylthio, C(H)=N-OH, C(H)=N-O(C₁₋₆ alkyl), C(C₁₋₆ alkyl)=N-OH and C(C₁₋₆ alkyl)=N-O-(C₁₋₆ alkyl), and wherein two substituents on adjacent carbon atoms of the 5-6 membered heterocyclic ring together may form a group -CR^{6a} -CR^{6a} =CR^{6a} -CR^{6a} -, wherein each R^{6a} independently is selected from hydrogen, halogen, cyano, nitro, C₁₋₄ alkyl, C₁₋₄ haloalkyl, C₁₋₄ alkoxy, C₁₋₄ haloalkoxy, C₁₋₄ haloalkylthio, C(H)=N-OH, C(H)=N-O(C₁₋₆ alkyl), C(C₁₋₆ alkyl)=N-OH and C(C₁₋₆ alkyl)=N-O-(C₁₋₆ alkyl) ;
or R⁶ is an aliphatic saturated or unsaturated group containing 3 to 13 carbon atoms and at least one silicon atom, wherein the aliphatic group may contain 1 to 3 heteroatoms, each heteroatom independently selected from oxygen, nitrogen and sulphur, and wherein the aliphatic group may be substituted by 1 to 4 independently selected halogen atoms ;
or R⁶ is (CR^{a} R^{b})ₘ-Cy-(CR^{c} R^{d})ₙ-Y₁ ;
or R⁶ is C₁₋₆ alkoxy, C₁₋₆ haloalkoxy, C₂₋₆ alkenyloxy, C₂₋₆ haloalkenyloxy, C₂₋₆ alkinyloxy, C₃₋₆ cycloalkyloxy, C₁₋₄ alkyl-C₃₋₇ cycloalkyloxy, C₅₋₇ cycloalkenyloxy or C₁₋₄ alkyl-C₅₋₇ cycloalkenyloxy ;
Z is C₁₋₄ alkylene ;
p is 0 or 1;
R²⁵ is hydrogen, halogen, C₁₋₄ alkyl, C₁₋₄ haloalkyl, C₁₋₄ alkoxy(C₁₋₄)alkyl, C₁₋₄ haloalkoxy (C₁₋₄) alkyl or Si(C₁₋₄ alkyl)₃ ;
R²⁶ and R²⁷ are each, independently, hydrogen, halogen, C₁₋₄ alkyl or C₁₋₄ haloalkyl;
R²⁵ is hydrogen, C₁₋₄ alkyl or C₁₋₄ haloalkyl;
R^{a}, R^{b}, R^{c} and R^{d} are each, independently, hydrogen or a C₁₋₄ alkyl group, which may substituted by 1 to 6 substituents, each substituent independently selected from halogen, hydroxy, cyano, carboxyl, methoxycarbonyl, ethoxycarbonyl, methoxy, ethoxy, methylsulfonyl, ethylsulfonyl, difluoromethoxy, trifluoromethoxy, trifluoromethylthio and trifluorothiomethoxy ;
Cy is a carbocyclic or heterocyclic 3-7 membered ring, which may be saturated, unsaturated or aromatic and which may contain a silicon atom as a ring member, wherein (CR^{a} R^{b})ₘ and (CR^{c}R^{d})ₙ may be bound either to the same carbon or silicon atom of Cy or to different atoms separated by 1, 2 or 3 ring members, wherein the carbocyclic or heterocyclic 3-7 membered ring may substituted by 1 to 6 substituents, each substituent independently selected from halogen, C₁₋₄ alkyl, C₂₋₄ alkenyl, C₁₋₄ haloalkyl, C₁₋₄alkoxy and halo-C₁₋₄ alkoxy ;
Y₁ is Si(Oₚ₁Z¹)(O_{q}Z²)(O≤Z³) and provided that Cy contains a silicon atom as a ring member then Y₁ may also be hydrogen ;
Z¹ and Z² are independently methyl or ethyl;
Z³ is a C₁₋₄ alkyl or a C₂₋₄ alkenyl group, which may be interrupted by one heteroatom selected from O, S and N, and wherein the C₁₋₄ alkyl or C₂₋₄ alkenyl group may be substituted by 1 to 3 independently selected halogen atoms ;
m and n are each independently 0, 1, 2 or 3 ;
p₁, q and s are each independently 0 or 1 ;
R⁷, R⁸, R⁹, R¹⁰, R¹¹, R¹² and R^{12a} are each, independently, hydrogen, halogen, cyano, nitro, C₁₋₄ alkyl, C₁₋₄ haloalkyl, C₁₋₄ alkoxy, C₁₋₄ haloalkoxy, C₁₋₄ thioalkyl or C₁₋₄ thiohaloalkyl ;
R¹³, R¹⁴, R¹⁵, R¹⁶ and R¹⁷ are each, independently, hydrogen, halogen, cyano, nitro, C₁₋₄ alkyl, C(O)CH₃, C₁₋₄ haloalkyl, C₁₋₄ alkoxy, C₁₋₄ haloalkoxy, C₁₋₄ thioalkyl, C₁₋₄ thiohaloalkyl, hydroxymethyl or C₁₋₄ alkoxymethyl ;
W is a single or a double bond ; and
Y is O, N(R¹⁸), S or (CR¹⁹R²⁰)(CR²¹R²²)m1(CR²³R²⁴)n1 ;
R¹⁸ is hydrogen, C₁₋₄ alkyl, formyl, C₁₋₄ alkoxy(C₁₋₄) alkyl, C(=O)C₁₋₄ alkyl, which may be substituted by halogen or C₁₋₄ alkoxy, or C(=O)O-C₁₋₆ alkyl, which may be substituted by halogen, C₁₋₄ alkoxy or CN ;
R¹⁹, R²⁰, R²¹, R²², R²³ and R²⁴ are each independently hydrogen, halogen, hydroxy, C₁₋₄ alkoxy, C₁₋₆ alkyl, which may be substituted by 1 to 3 substituents selected from halogen, hydroxy, =O, C₁₋₄ alkoxy, O-C(O)-C₁₋₄ alkyl, phenyl, naphthyl, anthracyl, fluorenyl, indanyl or a 3-7 membered carbocyclic ring (which itself may be substituted by 1 to 3 methyl groups), C₁₋₆ alkenyl, which may be substituted by 1 to 3 substituents selected from halogen, hydroxy, =O, C₁₋₄ alkoxy, O-C(O)-C₁₋₄ alkyl, phenyl, naphthyl, anthracyl, fluorenyl, indanyl or a 3-7 membered carbocyclic ring (which itself may be substituted by 1 to 3 methyl groups), or a 3- 7 membered carbocyclic ring, which may contain 1 heteroatom selected from nitrogen and oxygen, and wherein the 3-7 membered carbocyclic ring may be substituted by 1 to 3 methyl groups ;
or R¹⁹, R²⁰ together with the carbon atom to which they are attached form a carbonyl-group, a 3-5 membered carbocyclic ring, which may be substituted by 1 to 3 methyl groups, C₁₋₆ alkylidene, which may be substituted by 1 to 3 methyl groups, or C₃₋₆ cycloalkylidene, which may be substituted by 1 to 3 methyl groups ;
m₁ is 0 or 1;
n₁ is 0 or 1;
R^{13a} is a C₁-C₄ alkyl, C₂-C₄ alkenyl or C₂-C₄ alkynyl group, which may be substituted by 1 to 6 substituents, each substituent independently selected from halogen, hydroxy, cyano, C₁₋₄ alkoxycarbonyl, formyl, nitro, C₁-C₄ alkoxy, C₁-C₄ haloalkoxy, C₁-C₄ alkylthio, C₁-C₄ haloalkylthio, HC(OR²⁹)=N- and R³⁰R³¹NN=C(H)- ;
R²⁹, R³⁰ and R³¹ independently of one another are hydrogen or C₁-C₄ alkyl; R^{13b} is a C₁-C₆ alkyl group, which may be substituted by 1 to 6 substituents, each substituent independently selected from halogen, hydroxy, cyano, C₁₋₄ alkoxycarbonyl, formyl, nitro, C₁-C₄ alkoxy, C₁-C₄ haloalkoxy, C₁-C₄ alkylthio, C₁-C₄ haloalkylthio, HC(OR³²)=N- and R³³R³⁴NN=C(H)- ;
R³², R³³ and R²⁴ independently of one another are hydrogen or C₁-C₄ alkyl;
R^{13c} is hydrogen or halogen ; and tautomers/isomers/enantiomers of these compounds
which comprises reacting a compound of formula (II) 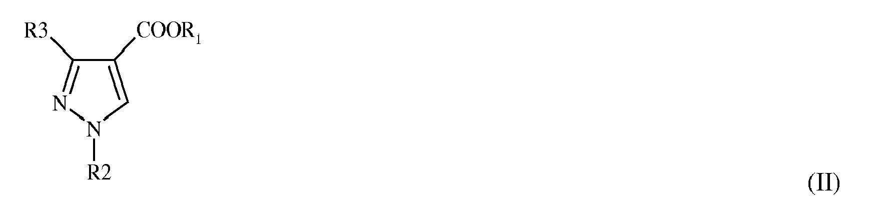 wherein
- R1 is an organic residue
- R2 and R3 are as defined above,
with an amine of formula (III) : QNHR4 (III) wherein Q and R4 are as defined above, in the presence of a Lewis acid.

2. The process according to claim 1, wherein the process is further carried out in the presence of an additional base which is different from the compound according to formula (III).

3. The process according claim 1 or 2, wherein the Lewis acid is selected from a group consisting of aluminium compounds and boron compounds, in particular aluminium halides and boron halides, more particularly aluminium trichloride and boron trichloride; and trialkylaluminum compounds and trialkylboron compounds, in particular trimethylaluminum.

4. The process according to any one of claims 2 to 4, wherein the additional base is a nucleophilic base, in particular wherein the nucleophilic base is selected from the group consisting of diethylamine, triethylamine, methylpiperidine and N-methylmorpholine, wherein triethylamine is the most preferred nucleophilic base.

5. The process according to claim 1 or 3, wherein the Lewis acid is present in an amount of from 0.25 to 4 molar equivalents relative to the amount of the compound of formula (II), preferably in an amount of from 0.75 to 2 molar equivalents relative to the amount of the compound of formula (II).

6. The process according to anyone of claims 2 to 4, wherein the Lewis acid is present in an amount of from 0.25 to 8 molar equivalents relative to the amount of the compound of formula (II), preferably in an amount of from 1 to 6 molar equivalents relative to the amount of the compound of formula (II).

7. The process according to anyone of claims 2, 3, 4 and 6, wherein the additional base is present in an amount of from 0.25 to 8 molar equivalents relative to the amount of the Lewis acid, preferably in an amount of from 1 to 6 molar equivalents relative to the amount of the Lewis acid.

8. The process according to any one of claims 1 to 7, wherein the reaction is carried out in an inert solvent selected from a group consisting of hydrocarbons, halogenated hydrocarbons, halogenated aromatic hydrocarbons, straight chain or cyclic ethers, or nitriles; preferably from halogenated hydrocarbons and straight chain or cyclic ethers; more preferably from straight chain or cyclic ethers.

9. The process according to claim 8, wherein the inert solvent is a cyclic ether, preferably dioxane.

10. The process according to any one of claims 1 to 9, wherein R3 is an alkyl group or a halogenated alkyl group ; preferably a fluorinated alkyl group selected from the group consisting of fluoromethyl, difluoromethyl, trifluoromethyl, chlorofluoromethyl, dichlorofluoromethyl, and chlorodifluoromethyl ; more preferably difluoromethyl.

11. The process according to anyone of claims 1 to 10, wherein R4 is selected from the group consisting of H, C₁-C₈-alkyl, benzyl and phenyl; in particular H, C₁-C₄-alkyl and benzyl; more particularly from H, methyl, ethyl, isopropyl and benzyl; especially H.

12. The process according to any one of claims 1 to 11, wherein Q is a group of formula Q1 or Q37.

13. The process according to any one of claims 1 to 11, wherein Q is a group of formula Q39 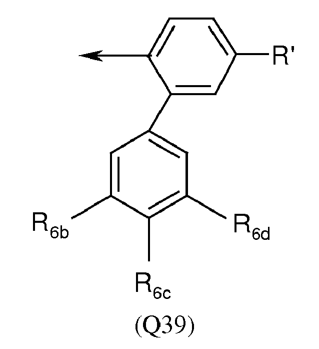 wherein R', R_{6b}, R_{6c} and R_{6d} are each, independently, hydrogen or halogen, said halogen is especially chlorine or fluorine.

14. The process according to any one of claims 1 to 11, Q is a group of formula Q40

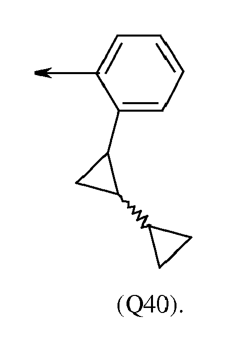

15. The process according to any one of claims 1 to 11, Q is a group of formula Q41

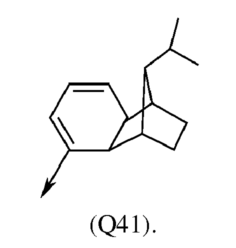

16. A process for the manufacture of pharmaceutically active or agrochemically active compounds or their precursors, comprising at least the process according to any one of claims 1 to 15.
